(19) Europäisches Patentamt
European Patent Office
Office européen des brevets

(11) Veröffentlichungsnummer: **0 173 313**
**A2**

# (12) EUROPÄISCHE PATENTANMELDUNG

(21) Anmeldenummer: **85110824.1**

(22) Anmeldetag: **19.08.85**

(51) Int. Cl.4: **C 07 D 239/42**, C 07 D 239/52, C 07 D 239/46, C 07 D 239/48, C 07 D 251/16, C 07 D 251/18, C 07 D 251/40, A 01 N 47/28, A 01 N 47/34

(30) Priorität: **30.08.84 DE 3431933**

(43) Veröffentlichungstag der Anmeldung: **05.03.86 Patentblatt 86/10**

(84) Benannte Vertragsstaaten: **CH DE FR GB IT LI**

(71) Anmelder: **BAYER AG, Konzernverwaltung RP Patentabteilung, D-5090 Leverkusen 1 Bayerwerk (DE)**

(72) Erfinder: **Diehr, Hans-Joachim, Dr., Höhe 35, D-5600 Wuppertal 1 (DE)**
Erfinder: **Fest, Christa, Dr., Im Johannistal 20, D-5600 Wuppertal 1 (DE)**
Erfinder: **Kirsten, Rolf, Dr., Carl-Langhans-Strasse 27, D-4019 Monheim (DE)**
Erfinder: **Kluth, Joachim, Dr., Kurt-Schumacher-Strasse 9, D-4018 Langenfeld (DE)**
Erfinder: **Müller, Klaus-Helmut, Dr., Bockhackstrasse 55, D-4000 Düsseldorf 13 (DE)**
Erfinder: **Pfister, Theodor, Dr., Lichtenberger Strasse 30, D-4019 Monheim (DE)**
Erfinder: **Priesnitz, Uwe, Dr., Severinstrasse 58, D-5650 Solingen 1 (DE)**
Erfinder: **Riebel, Hans-Jochem, Dr., In der Beek 92, D-5600 Wuppertal 1 (DE)**
Erfinder: **Roy, Wolfgang, Dr., Walter-Kolb-Strasse 47, D-4018 Langenfeld (DE)**
Erfinder: **Santel, Hans-Joachim, Dr., Gerstenkamp 19, D-5000 Koeln 80 (DE)**
Erfinder: **Schmidt, Robert, R., Dr., Im Waldwinkel 110, D-5060 Bergisch-Gladbach 2 (DE)**

(54) **Heteroarylthioharnstoffe.**

(57) Die Erfindung betrifft neue Heteroarylthioharnstoffe der allgemeinen Formel (I)

$$R-NH-\overset{S}{\overset{\|}{C}}-NH-R^1 \quad (I)$$

(worin die Reste R und $R^1$ die in der Beschreibung angegebenen Bedeutung haben),

Verfahren zu ihrer Herstellung und ihre Verwendung als Herbizide und als Zwischenprodukt zur Herstellung von Herbiziden.

EP 0 173 313 A2

BAYER AKTIENGESELLSCHAFT 5090 Leverkusen, Bayerwerk
Konzernverwaltung RP Bi/O
Patentabteilung Ib

# Heteroarylthioharnstoffe

Die Erfindung betrifft neue Heteroarylthioharnstoffe, Verfahren zu ihrer Herstellung, ihre Verwendung als Herbizide und als Zwischenprodukte für Herbizide.

Es ist bekannt, daß bestimmte Heteroarylthioharnstoffe, wie z. B. 4-Methoxy-6-methyl-pyrimidin-2-yl-thioharnstoff, herbizid wirksam sind. Die Wirkung dieser Verbindungen ist jedoch nicht immer ganz befriedigend (vergl. EP-OS 117 014).

Es wurden nun neue Heteroarylthioharnstoffe der allgemeinen Formel (I)

$$R\text{-}NH\text{-}\overset{\overset{S}{\|}}{C}\text{-}NH\text{-}R^1 \qquad (I)$$

in welchen

$R^1$ für den Rest 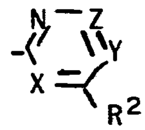 steht, worin

$R^2$ für Wasserstoff, Halogen, Hydroxy, Amino, Alkylamino, Di-alkylamino sowie für gegebenenfalls substituierte Reste aus der Reihe Alkyl, Alkoxy oder Alkylthio steht,

Y für Stickstoff oder für den Rest $-CR^3=$ steht, wobei $R^3$ für Wasserstoff, Halogen oder Alkyl steht,

X für Stickstoff und - für den Fall, daß Y für Stickstoff steht - außerdem für einen Methin-Rest $-CH=$ steht,

Z für Stickstoff oder für den Rest $-CR^4=$ steht, wobei $R^4$ für Wasserstoff, Halogen, Hydroxy, Amino, Alkylamino, Di-alkylamino sowie gegebenenfalls substituierte Reste aus der Reihe Alkyl, Alkoxy oder Alkylthio steht und

R für Wasserstoff oder für den Rest $-CO-R^5$ steht, wobei

$R^5$ für gegebenenfalls durch Halogen, Phenyl und/oder $C_1$-$C_4$-Alkoxy substituiertes Alkyl, für gegebenenfalls durch Halogen, Phenyl und/oder $C_1$-$C_4$-Alkoxy substituiertes Alkoxy, für Alkenyl, Alkinyl, sowie für gegebenenfalls durch $C_1$-$C_4$-Alkyl, $C_1$-$C_4$-Alkylthio, Halogen-$C_1$-$C_4$-Alkyl, Halogen-$C_1$-$C_4$-Alkoxy, Halogen-$C_1$-$C_4$-Alkylthio, Fluor, Chlor, Brom, Cyano, Nitro und/oder $C_1$-$C_4$-Alkoxycarbonyl sub-

stituiertes Phenyl steht,gefunden, wobei die folgenden Verbindungen ausgenommen sind:

4,6-Dimethylpyrimidin-2-yl-thioharnstoff;

1-(Ethoxycarbonyl)-3-(4,6-dimethylpyrimidin-2-yl)-thioharnstoff;

1-(Ethoxycarbonyl)-3-(pyrimidin-2-yl-)thioharnstoff;
2,4-Dimethylpyrimidin-6-yl-thioharnstoff und
1-(Ethoxycarbonyl)-3-(2,4-dimethyl-pyrimidin-6-yl)-thioharnstoff ; 4-Methoxy-6-methyl-pyrimidin-2-yl-thioharnstoff und 1-(Benzoyl)-3-(4-methoxy-6-methyl-pyrimidin-2-yl)-thioharnstoff.

Man erhält die neuen Verbindungen der Formel (I), wenn man

(a) für den Fall, daß R für Wasserstoff steht, Heteroarylthioharnstoffe der Formel (Ia)

$$R^5-\overset{O}{\overset{\|}{C}}-NH-\overset{S}{\overset{\|}{C}}-NHR^1 \qquad (Ia)$$

in welcher

$R^1$ und $R^5$ die oben angegebenen Bedeutungen haben, mit wäßrigen Basen gegebenenfalls in Gegenwart von Verdünnungsmitteln umsetzt und gegebenenfalls die hierbei erhaltenen Hydrolyseprodukte mit Säuren behandelt, oder

(b) für den Fall, daß R für den Rest $-\overset{O}{\overset{\|}{C}}R^5$ steht, Amino-hetarene der Formel (II)

$$H_2N-R^1 \qquad (II)$$

in welcher

$R^1$ die oben angegebene Bedeutung hat,

mit Carbonylisothiocyanaten der Formel (III)

$$R^5-\overset{\overset{O}{\|}}{C}-N=C=S \qquad (III)$$

in welcher

$R^5$ die oben angegebene Bedeutung hat,
gegebenenfalls in Gegenwart von Verdünnungsmitteln umsetzt.

Die neuen Heteroarylthioharnstoffe der Formel (I) zeichnen sich durch starke herbizide Wirksamkeit aus.

Ueberraschenderweise zeigen die neuen Verbindungen der Formel (I) erheblich stärkere herbizide Wirkung als viele bekannte chemische Verbindungen gleicher Wirkungsrichtung.

Die Verbindungen der Formel (I), in welcher R für Wasserstoff steht, können als Zwischenprodukte für die Herstellung von Heteroaryl-cyanamiden der Formel $R^1$-NH-CN verwendet werden (vgl. DE-OS 3 334 455).

Gegenstand der Erfindung sind vorzugsweise Verbindungen der Formel (I), in welcher

$R^1$ für den Rest 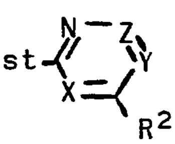 steht, worin

$R^2$ vorzugsweise für Wasserstoff, Fluor, Chlor, Brom, Hydroxy, $C_1$-$C_4$-Alkyl [welches gegebenenfalls durch Fluor und/oder Chlor substituiert ist], $C_1$-$C_4$-Alkoxy [welches gegebenenfalls durch Fluor

und/oder Chlor substituiert ist], $C_1$-$C_4$-Alkylthio [welches gegebenenfalls durch Fluor und/oder Chlor substituiert ist], Amino, $C_1$-$C_4$-Alkylamino oder Di-($C_1$-$C_4$-alkyl)-amino steht,

Y für Stickstoff oder für den Rest $-CR^3=$ steht, wobei $R^3$ vorzugsweise für Wasserstoff, Chlor, Fluor, Brom oder $C_1$-$C_4$-Alkyl steht,

X für Stickstoff und - für den Fall, daß Y für Stickstoff steht - außerdem für einen Methin-Rest $-CH=$ steht,

Z für Stickstoff oder für den Rest $-CR^4=$ steht, wobei $R^4$ vorzugsweise für Wasserstoff, Fluor, Chlor, Brom, Hydroxy, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio [welches gegebenenfalls durch Fluor und/oder Chlor substituiert ist], Amino, $C_1$-$C_4$-Alkylamino oder Di($C_1$-$C_4$-alkyl)amino steht und

R für Wasserstoff oder für den Rest $-CO-R^5$ steht, worin $R^5$ vorzugsweise für gegebenenfalls durch Fluor, Chlor, Brom, Phenyl und/oder $C_1$-$C_2$-Alkoxy substituiertes $C_1$-$C_6$-Alkyl, für gegebenenfalls durch Fluor, Chlor, Brom, Phenyl und/oder $C_1$-$C_2$-Alkoxy substituiertes $C_1$-$C_6$-Alkoxy, für $C_3$-$C_6$-Alkenyl, $C_3$-$C_6$-Alkinyl sowie für gegebenenfalls durch Methyl, Ethyl,

Methoxy, Ethoxy, Methylthio, Ethylthio, Trifluormethyl, Difluormethyl, Trifluormethoxy, Difluormethoxy, Fluor, Chlor, Cyano, Nitro und/oder $C_1$-$C_2$-Alkoxycarbonyl substituiertes Phenyl steht, wobei die bei der allgemeinen Restedefinition der Formel (I) genannten Verbindungen ausgenommen sind.

Gegenstand der Erfindung sind insbesondere Verbindungen der Formel (I), in welcher

$R^1$ für den Rest 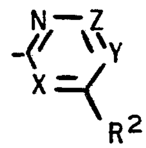 steht, worin

$R^2$ für Chlor, Methyl, Methoxy oder Ethoxy steht,

Y für einen Methin-Rest -CH= steht,

X für Stickstoff steht,

Z für den Rest -$CR^4$= steht, wobei

$R^4$ für Wasserstoff, Chlor, Methyl, Methoxy, Ethoxy, Methylthio, Ethylthio, Dimethylamino oder Diethylamino steht und

R für den Rest -CO-$R^5$ steht, worin

$R^5$ für Methoxy, Ethoxy, n-Propoxy, i-Propoxy, n-Butoxy, i-Butoxy, sec. Butoxy, tert.-Butoxy sowie für gegebenenfalls durch Methyl, Ethyl, Methoxy, Ethoxy, Methylthio, Ethylthio, Trifluormethyl, Difluormethyl, Difluormethoxy, Fluor, Chlor, Brom,

Cyano, Nitro, Methoxycarbonyl und/oder Ethoxycarbonyl substituiertes Phenyl steht,

wobei die bei der allgemeinen Restdefinition der Formel (I) genannten Verbindungen ausgenommen sind.

Verwendet man 1-(Methoxycarbonyl)-3-(4-methyl-6-methoxypyrimidin-2-yl)-thioharnstoff als Ausgangsstoff, und wäßrige Natriumhydroxidlösung als Base und säuert anschließend mit Salzsäure an, so kann der Verlauf des erfindungsgemäßen Verfahrens (a) durch das folgende Formelschema wiedergegeben werden:

(a)

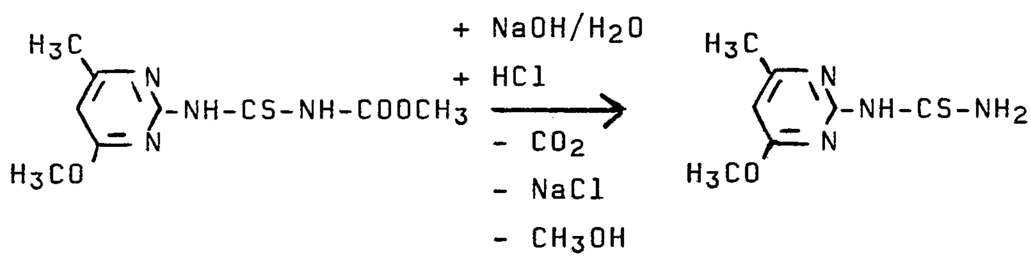


Verwendet man 2-Amino-4,6-dimethoxy-pyrimidin und Phenylcarbonylisothiocyanat als Ausgangsstoffe, so kann der Verlauf des erfindungsgemäßen Verfahrens (b) durch das folgende Formelschema wiedergegeben werden:

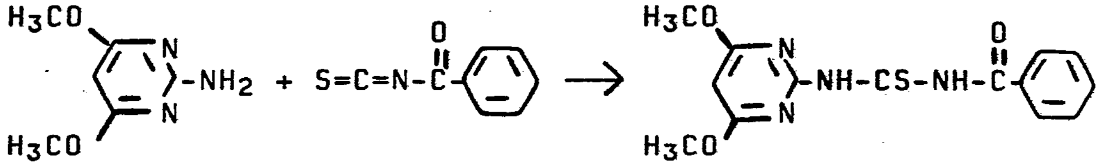

(a)

Die beim erfindungsgemäßen Herstellungsverfahren (a) als Ausgangsstoffe zu verwendenden Heteroarylthioharnstoffe der Formel (Ia) sind erfindungsgemäße Verbindungen.
In dieser Formel haben $R^1$ und $R^5$ vorzugsweise bzw. insbesondere die gleichen Bedeutungen, wie sie im Rahmen der Definitionen der entsprechenden Reste in Formel (I) vorzugsweise bzw. als insbesondere bevorzugt genannt wurden.

Man erhält die Verbindungen der Formel (Ia) nach dem erfindungsgemäßen Herstellungsverfahren (b) . Die beim erfindungsgemäßen Verfahren (b) als Ausgangsstoffe zu verwendenden Amino-hetarene sind durch die Formel (II) allgemein definiert. In dieser Formel hat $R^1$ vorzugsweise bzw. insbesondere die gleichen Bedeutungen, wie sie oben im Rahmen der Definition des entsprechenden Restes in Formel (I) vorzugsweise bzw. als insbesondere bevorzugt genannt wurden.

Die Ausgangsstoffe der Formel (II) sind bekannt und/oder können nach an sich bekannten Verfahren hergestellt werden (vgl. Chem. Pharm. Bull. 11 , 1382-1388 (1963); J. Chem. Soc. 1946 , 81 und US-PS 4299 960).

Die beim erfindungsgemäßen Verfahren (b) weiterhin als Ausgangsstoffe zu verwendenden Carbonylisothiocyanate sind durch die Formel (III) allgemein definiert. In dieser Formel hat $R^5$ vorzugsweise bzw. insbesondere die gleichen Bedeutungen, wie sie oben im Rahmen der Definitionen des entsprechenden Restes in Formel (I) vorzugsweise bzw. als insbesondere bevorzugt genannt wurden. Die Verbindungen der Formel (III) sind bekannt und/oder können nach an sich bekannten Verfahren hergestellt werden (vgl. J. Heterocycl. Chem. 5 , 837 (1968) und US-PS 4.160.037).

Das erfindungsgemäße Verfahren (a) wird vorzugsweise unter Verwendung von Wasser als Verdünnungsmittel durchgeführt. Als weitere Verdünnungsmittel kommen praktisch alle inerten organischen, vorzugsweise aprotisch polare Solventien in Betracht. Hierzu gehören gegebenenfalls halogenierte Kohlenwasserstoffe, wie z.B. Methylenchlorid, Chloroform, 1,2-Dichlorethan, Toluol, Xylol und Chlorbenzol, Nitrile, wie z.B. Acetonitril und Propionitril, Ether, wie z.B. 1,2-Dimethoxyethan, Tetrahydrofuran und Dioxan, sowie Dimethylformamid,Dimethylacetamid, Dimethylsulfoxid, Sulfolan, Pyridin und 2-Methyl-5-ethylpyridin.
Als Basen können bei Verfahren (a) praktisch alle üblicherweise verwendeten Säurebindemittel eingesetzt werden. Hierzu gehören insbesondere Alkalimetall- und Erdalkalimetallhydroxide.

Die Reaktionstemperaturen können beim erfindungsgemäßen Verfahren (a) innerhalb eines größeren Bereiches variiert werden. Im allgemeinen arbeitet man zwischen 0°C und +120°C, vorzugsweise zwischen 20°C und 100°C. Das erfindungsgemäße Verfahren (a) wird im allgemeinen bei Normaldruck durchgeführt.

Die bei Verfahren (a) gebildeten Produkte werden mit Säuren behandelt. Es kommen verschiedenste Protonendonatoren in Betracht, wie z.B. Salzsäure, Schwefelsäure und Phosphorsäure.

Zur Durchführung des erfindungsgemäßen Verfahrens (a) setzt man je Mol Verbindung der Formel (Ia) im allgemeinen zwischen 1 und 10 Mol, vorzugsweise zwischen 5 und 8 Mol einer Base in Form einer wäßrigen Lösung ein.

Die Reaktionskomponenten werden gewöhnlich bei Raumtemperatur oder unter Außenkühlung zusammengegeben und das Reaktionsgemisch wird bis zum Reaktionsende gerührt.

Die Aufarbeitung der Reaktionsprodukte kann auf übliche Weise durchgeführt werden, z.B. durch Absaugen des ausgefallenen Produktes. Zur Charakterisierung dient der Schmelzpunkt.

Das erfindungsgemäße Verfahren (b) wird vorzugsweise unter Verwendung von Verdünnungsmitteln durchgeführt. Als Verdünnungsmittel kommen praktisch alle inerten

organischen Solventien in Betracht. Hierzu gehören gegebenenfalls halogenierte Kohlenwasserstoffe, wie z.B. Methylenchlorid, Chloroform, Toluol und Chlorbenzol, Ether, wie z.B. Diethylether, Tetrahydrofuran, Dioxan und 1,2-Dimethoxyethan, Nitrile, wie z.B. Acetonitril und Propionitril sowie Dimethylformamid,Dimethylacetamid, Dimethylsulfoxid und Sulfolan.

Die Reaktionstemperaturen können beim erfindungsgemäßen Verfahren (b) innerhalb eines größeren Bereiches variiert werden. Im allgemeinen arbeitet man bei Temperaturen zwischen 0°C und 150°C, vorzugsweise zwischen 10°C und 100°C. Das erfindungsgemäße Verfahren (b) wird im allgemeinen bei Normaldruck durchgeführt.

Zur Durchführung des erfindungsgemäßen Verfahrens (b) werden die Ausgangsstoffe der Formeln(II) und (III) gewöhnlich in etwa äquimolaren Mengen eingesetzt. Ein Ueberschuß der einen oder anderen Reaktionskomponente bringt keine wesentlichen Vorteile.

Die Aufarbeitung der Reaktionsprodukte geschieht nach üblichen Methoden, z.B. durch Absaugen des ausgefallenen Produktes. Zur Charakterisierung dient der Schmelzpunkt.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. N-(2-Benzthiazolyl)-N,N'-dimethyl-harnstoff, 3-(3-Chlor-4-methylphenyl)-1,1-dimethylharnstoff, 3-(4-isopropylphenyl)-1,1-dimethylharnstoff, 4-Amino-6-(1,1-dimethylethyl)-3-methylthio-1,2,4-triazin-5(4H)-on, 4-Amino-6-(1,1-dimethyl-ethyl)-3-ethylthio-1,2,4-triazin-5(4H)-on, 1-Amino-6-ethylthio-3-(2,2-dimethylpropyl)-1,3,5-triazin-2,4-(1H, 3H)-dion, 4-Amino-3-methyl-6-phenyl-1,2,4-triazin-5(4H)-on, 2-Chlor-4-ethylamino-6-isopropyl-amino-1,3,5-triazin, das R-Enantiomere des 2-/4-(3,5-Dichlor-pyridin-2-oxy)-phenoxy/-propionsäure-(trimethylsilyl)-methylesters, das R-Enantiomere des 2-/4-(3,5-Dichlorpyridyl-2-oxy)-phenoxy/-propionsäure-(2-benzyloxy)-ethylesters, 2,4-Dichlorphenoxyessigsäure, 2-(2,4-Dichlorphenoxy)-propionsäure, 4-Chlor-2-methyl-phenoxy-essigsäure, 2-(2-Methyl-4-chlor-phenoxy)-propionsäure, 3,5-Dijod-4-hydroxy-benzonitril, 3,5-Dibrom-4-hydroxy-benzonitril sowie Diphenylether und Phenylpyridazine, wie z.B. Pyridate. Einige Mischungen zeigen überraschenderweise auch synergistische Wirkung.

Auch eine Mischung mit anderen bekannten Wirkstoffen, wie fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1

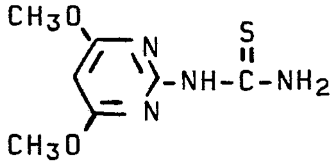

(Verfahren (a) ):

Eine Mischung von 5,0 g (0,0175 Mol) 1-(Ethoxycarbonyl)-3-(4,6-dimethoxy-pyrimidin-2-yl)-thioharnstoff, 4,0 g (0,1 Mol) Natriumhydroxid und von 100 ml Wasser wird 2 Tage bei 20°C gerührt. Dann wird unter Rühren solange verdünnte Salzsäure zugetropft, bis die Lösung sauer gestellt ist und die $CO_2$-Entwicklung beendet ist. Das kristallin angefallene Produkt wird durch Absaugen isoliert.

Man erhält 3,5 g (94 % der Theorie) 4,6-Dimethoxypyrimidin-2-yl-thioharnstoff vom Schmelzpunkt 245-8°C (Zers.).

Beispiel 2

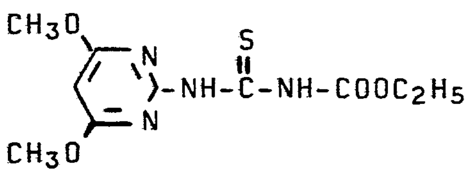

Verfahren (b):

Eine Mischung aus 15,5 g (0,1 Mol) 2-Amino-4,6-dimethoxy-pyrimidin, 13,1 g (0,1 Mol) Ethoxycarbonylisothiocyanat und 200 ml Acetonitril wird 2 Stunden bei 60°C gerührt. Dann wird auf 10°C abgekühlt, und das kristallin angefallene Produkt durch Absaugen isoliert.

Man erhält 22,5 g (79 % der Theorie) 1-(Ethoxycarbonyl)-3-(4,6-dimethoxy-pyrimidin-2-yl)-thioharnstoff vom Schmelzpunkt 194°C (Zers.).

Analog Beispiel 1 und 2 bzw. Verfahren (a) und (b) können die in der Tabelle 1 und 2 aufgeführten Verbindungen der Formel (I) bzw. (Ia) hergestellt werden:

$$R-NH-\overset{S}{\overset{\|}{C}}-NH-R^1 \qquad (I)$$

Tabelle 1

| Beispiel Nr. | R | $R^1$ | Schmelzpunkt (°C) |
|---|---|---|---|
| 3 | H | 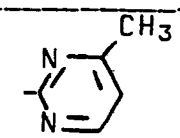 | 264-265 (Zers.) |

Tabelle 1 - Fortsetzung

| Beispiel Nr. | R | $R^1$ | Schmelzpunkt (°C) |
|---|---|---|---|
| 4 | H | 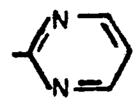 | 263 |
| 5 | H | 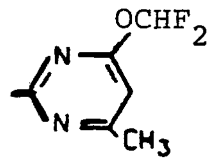 | 192-194 |
| 6 | H | 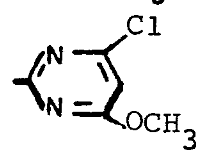 | 225-227 |
| 7 | H | 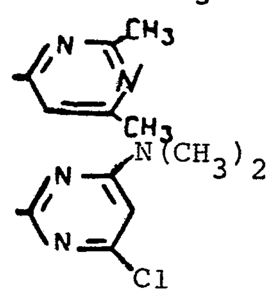 | 248 |
| 8 | H | | |
| 9 | H | 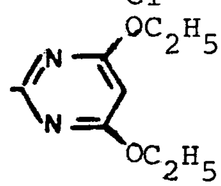 | 166 |

$$R^5-CO-NH-\overset{S}{\overset{\|}{C}}-NH-R^1 \qquad (Ia)$$

Tabelle 2

| Beispiel Nr. | $R^5$ | $R^1$ | Schmelzpunkt (°C) |
|---|---|---|---|
| 10 | 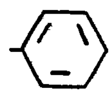 | 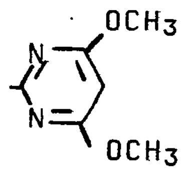 | 189 |
| 11 | 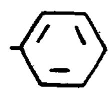 | 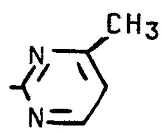 | 198-9 (Zers.) |
| 12 | $-OC_2H_5$ | 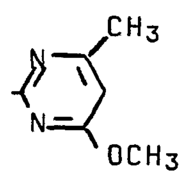 | 217 |

Tabelle 2 - Fortsetzung

| Beispiel Nr. | $R^5$ | $R^1$ | Schmelzpunkt (°C) |
|---|---|---|---|
| 13 | 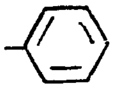 | 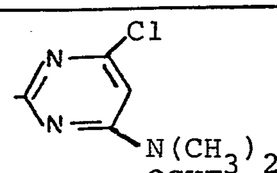 | 168 |
| 14 | 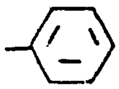 | 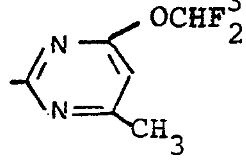 | 182 |
| 15 | $-OC_2H_5$ | 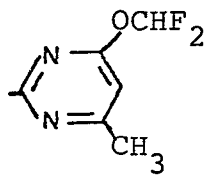 | 184-185 |
| 16 | $-OC_2H_5$ | 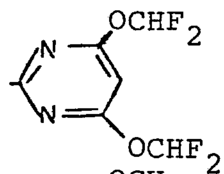 | 173 |
| 17 | $-OC_2H_5$ | 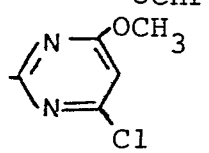 | 100 |
| 18 | 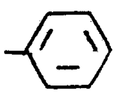 | 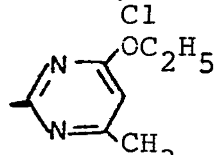 | 156 |
| 19 | 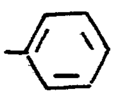 | 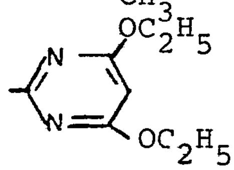 | 179 |
| 20 | $-OC_2H_5$ | 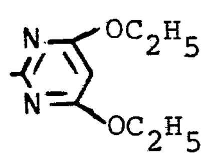 | 159 |
| 21 | 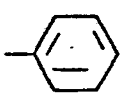 | 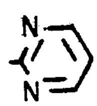 | 172-173 |

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Die erfindungsgemäßen Wirkstoffe zeigen in diesem Test eine sehr gute herbizide Wirksamkeit.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Die erfindungsgemäßen Wirkstoffe zeigen in diesem Test eine sehr gute herbizide Wirksamkeit.

## Patentansprüche

1) Heteroarylthioharnstoffe der allgemeinen Formel (I)

$$RHN-\overset{S}{\overset{\|}{C}}-NHR^1 \qquad (I)$$

in welcher

$R^1$ für den Rest 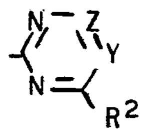 steht, worin

$R^2$ für Wasserstoff, Halogen, Hydroxy, Amino, Alkylamino, Di-alkylamino sowie für gegebenenfalls substituierte Reste aus der Reihe Alkyl, Alkoxy oder Alkylthio steht,

Y für Stickstoff oder für den Rest $-CR^3=$ steht, wobei $R^3$ für Wasserstoff, Halogen oder Alkyl steht,

X für Stickstoff und - für den Fall, daß Y für Stickstoff steht - außerdem für einen Methin-Rest -CH= steht,

Z für Stickstoff oder für den Rest $-CR^4=$ steht, wobei $R^4$ für Wasserstoff, Halogen, Hydroxy, Amino, Alkylamino, Di-alkylamino sowie gegebenenfalls substituierte Reste aus der Reihe Alkyl, Alkoxy oder Alkylthio steht und

R für Wasserstoff oder für den Rest $-CO-R^5$ steht,

wobei

$R^5$ für gegebenenfalls durch Halogen, Phenyl und/oder $C_1$-$C_4$-Alkoxy substituiertes Alkyl, für gegebenenfalls durch Halogen, Phenyl, und/oder $C_1$-$C_4$- Alkoxy substituiertes Alkoxy, für Alkenyl, Alkinyl, sowie für gegebenenfalls durch $C_1$-$C_4$-Alkyl,$C_1$-$C_4$ Alkylthio, Halogen-$C_1$-$C_4$-Alkyl, Halogen-$C_1$-$C_4$-Alkoxy, Halogen-$C_1$-$C_4$-Alkylthio, Fluor, Chlor,Brom, Cyano, Nitro und/oder $C_1$-$C_4$-Alkoxycarbonyl substituiertes Phenyl steht, wobei die folgenden Verbindungen ausgenommen sind:

4,6-Dimethylpyrimidin-2-yl-thioharnstoff;

1-(Ethoxycarbonyl)-3-(4,6-dimethylpyrimidin-2-yl)-thioharnstoff;

1-(Ethoxycarbonyl)-3-(pyrimidin-2-yl-)thioharnstoff;
2,4-Dimethylpyrimidin-6-yl-thioharnstoff und
1-(Ethoxycarbonyl)-3-(2,4-dimethyl-pyrimidin-6-yl)-thioharnstoff ; 4-Methoxy-6-methyl-pyrimidin-2-yl-thioharnstoff und 1-(Benzoyl)-3-(4-methoxy-6-methyl-pyrimidin-2-yl)-thioharnstoff.

2) Verfahren zur Herstellung von Heteroarylthioharnstoffen der allgemeinen Formel (I) gemäß Anspruch 1, dadurch gekennzeichnet, daß man

(a) für den Fall, daß R für Wasserstoff steht, Heteroarylthioharnstoffe der Formel (Ia)

$$R^5-\overset{O}{\overset{\|}{C}}-NH-\overset{S}{\overset{\|}{C}}-NHR^1 \qquad (Ia)$$

in welcher

$R^1$ und $R^5$ die oben angegebenen Bedeutungen haben, mit wäßrigen Basen gegebenenfalls in Gegenwart von Verdünnungsmitteln umsetzt und gegebenenfalls die hierbei erhaltenen Hydrolyseprodukte mit Säuren behandelt,

oder daß man

(b) für den Fall, daß R für den Rest $-\overset{\overset{O}{\|}}{C}R^5$ steht, Amino-hetarene der Formel (II)

$$H_2N-R^1 \quad (II)$$

in welcher

$R^1$ die oben angegebene Bedeutung hat, mit Carbonylisothiocyanaten der Formel (III)

$$R^5-\overset{\overset{O}{\|}}{C}-N=C=S \quad (III)$$

in welcher

$R^5$ die oben angegebene Bedeutung hat, gegebenenfalls in Gegenwart von Verdünnungsmitteln umsetzt.

3) Herbizide Mittel, gekennzeichnet durch einen Gehalt an mindestens einem Heteroarylthioharnstoff der allgemeinen Formel (I) gemäß Anspruch 1 .

4) Verwendung von Heteroarylthioharnstoffen der allgemeinen Formel (I) gemäß Anspruch 1 zur Bekämpfung von unerwünschtem Pflanzenwachstum.

5) Verfahren zur Herstellung von herbiziden Mitteln, dadurch gekennzeichnet, daß man Heteroarylthioharnstoffe der allgemeinen Formel (I) gemäß Anspruch 1 mit Streckmitteln und/oder oberflächenaktiven Mitteln vermischt.